# EUROPEAN PATENT SPECIFICATION

(11) **EP 1 917 936 B1**
(45) Date of publication and mention of the grant of the patent: **25.03.2009**
(21) Application number: 07115341.5
(22) Date of filing: 30.08.2007
(51) Int. Cl.: A61F 9/007

(54) **Ultrasound apparatus**
Ultraschallvorrichtung
Appareil à ultrasons

(30) Priority: 01.11.2006 US 591352
(43) Date of publication of application: 07.05.2008
(73) Proprietor: Alcon, Inc., 6331 Hünenberg (CH)
(72) Inventor: Chon, James Y., Irvine, CA 92604 (US); Gordon, Raphael, Ladera Ranch, CA 92694 (US)
(74) Representative: Hanna, Peter William Derek

(56) References cited:
- WO-A-00/00096
- US-A- 3 589 363
- US-A1- 2006 041 220

## Description

### Background of the Invention

This invention relates to ultrasonic devices and more particularly to torsional ultrasound ophthalmic phacoemulsification handpieces.

A typical ultrasonic surgical device suitable for ophthalmic procedures consists of an ultrasonically driven handpiece, an attached hollow cutting tip, an irrigating sleeve and an electronic control console. The handpiece assembly is attached to the control console by an electric cable and flexible tubings. Through the electric cable, the console varies the power level transmitted by the handpiece to the attached cutting tip and the flexible tubings supply irrigation fluid to and draw aspiration fluid from the eye through the handpiece assembly.

Although some prior art handpieces are of the magnetostrictive type, the operative part of most commercially available handpieces is a centrally located, and hollow resonating bar or horn directly attached to a set of piezoelectric crystals. The crystals supply the required ultrasonic vibration needed to drive both the horn and the attached cutting tip during phacoemulsification and are controlled by the console. The crystal/horn assembly is suspended within the hollow body or shell of the handpiece at its nodal points by relatively inflexible mountings. The handpiece body terminates in a reduced diameter portion or nosecone at the body's distal end. The nosecone is externally threaded to accept the irrigation sleeve. Likewise, the horn bore is internally threaded at its distal end to receive the external threads of the cutting tip. The irrigation sleeve also has an internally threaded bore that is screwed onto the external threads of the nosecone. The cutting tip is adjusted so that the tip projects only a predetermined amount past the open end of the irrigating sleeve.

When used to perform phacoemulsification, the ends of the cutting tip and irrigating sleeve are inserted into a small incision of predetermined width in the cornea, sclera, or other location in the eye tissue in order to gain access to the anterior chamber of the eye. The cutting tip is ultrasonically vibrated along its longitudinal axis within the irrigating sleeve by the crystal-driven ultrasonic horn, thereby emulsifying upon contact the selected tissue in situ. The hollow bore of the cutting tip communicates with the bore in the horn that in turn communicates with the aspiration line from the handpiece to the console. A reduced pressure or vacuum source in the console draws or aspirates the emulsified tissue from the eye through the open end of the cutting tip, the bore of the cutting tip, the horn bore, and the aspiration line and into a collection device. The aspiration of emulsified tissue is aided by a saline flushing solution or irrigant that is injected into the surgical site through the small annular gap between the inside surface of the irrigating sleeve and the outside surface of the cutting tip.

There have been prior attempts to combine ultrasonic longitudinal motion of the cutting tip with rotational motion of the tip, see U.S. Patent Nos. 5,222,959 (Anis), 5,722,945 (Anis, et al.) and 4,504,264 (Kelman). These prior attempts have used electric motors to provide the rotation of the tip which require O-ring or other seals that can fail in addition to the added complexity and possible failure of the motors. There have also been prior attempts to generate both longitudinal and torsional motion without the use of electric motors. For example, in U.S. Patent Nos. 6,028,387, 6,077,285 and 6,402,769 (Boukhny), one of the inventors of the current invention, describes a handpiece having two pairs of piezoelectric crystals. One pair is polarized to produce longitudinal motion. The other pair is polarized to produce torsional motion. Two separate drive signals are used to drive the two pairs of crystals. In actual practice, making a handpiece using two pairs of crystals resonate in both longitudinal and torsional directions is difficult to achieve. One possible solution, also described by one of the current inventors, is described in U.S. Patent Publication No. US 2001/0011176 A1 (Boukhny) and U.S. Patent Publication No. US 2006/0041200 A1 (Boukhny, et al.). These references disclose a handpiece having a single set of piezoelectric crystals that produce longitudinal motion, and a series of diagonal slits on the handpiece horn or tip that produce torsional motion when the horn or tip is driven at the resonate frequency of the piezoelectric crystals.

Accordingly, a need continues to exist for a reliable ultrasonic handpiece that will vibrate both longitudinally and torsionally, either simultaneously or separately, without the use of piezoelectric crystals.

### Brief Summary of the Invention

The present invention improves upon prior art ultrasonic handpieces by providing a magnetostrictive ultrasonic handpiece capable of providing both longitudinal and torsional or twisting motion, in accordance with claims which follow.

Accordingly, one objective of the present invention is to provide an ultrasound handpiece having both longitudinal and torsional motion.

Another objective of the present invention is to provide a magnetostrictive ultrasound handpiece having both longitudinal and torsional motion.

Other objects, features and advantages of the present invention will become apparent with reference to the drawings, and the following description of the drawings and claims.

### Brief Description of the Drawings

FIG. 1 is a perspective view of a handpiece and control console that may be used with the present invention.
FIG. 2 is a perspective view of a first embodiment of a magnetostrictive core that may be used with the present invention.
FIG. 3 is a perspective view of a first embodiment of a magnetostrictive core that may be used with the present invention similar to the magnetostrictive core illustrated in FIG. 2.
FIG. 4 is a perspective view of a second embodiment of a magnetostrictive core that may be used with the present invention.
FIG. 5 is a perspective view of a second embodiment of a magnetostrictive core that may be used with the present invention similar to the magnetostrictive core illustrated in FIG. 4.

### Detailed Description of the Invention

Magnetostriction (or the Joule effect) is a property of ferromagnetic materials that causes them to change their shape when subjected to a magnetic field. The effect was first identified in 1842 by James Joule when observing a sample of nickel. Internally, ferromagnetic materials have a crystal structure that is divided into domains, each of which is a region of uniform magnetic polarisation. When a magnetic field is applied, the boundaries between the domains shift and the domains rotate, both these effects causing a change in the material's dimensions.

An important characteristic of a wire made of a magnetostrictive material is the Wiedemann effect. When an axial magnetic field is applied to a magnetostrictive element, and a current is passed through the element, a twisting occurs at the location of the axial magnetic field. The twisting is caused by interaction of the axial magnetic field with the magnetic field along the magnetostrictive element, which is present due to the current in the element. The current is applied as a short-duration pulse, - I or 2 µs; the minimum current density is along the center of the element and the maximum at the element surface. The magnetic field intensity is also greatest at the element surface. This aids in developing the waveguide twist, as the current is applied as a pulse, the mechanical twisting travels in the element as an ultrasonic wave. The magnetostrictive element is therefore called the waveguide. The wave travels at the speed of sound in the waveguide material, ∼ 3000 m/s.

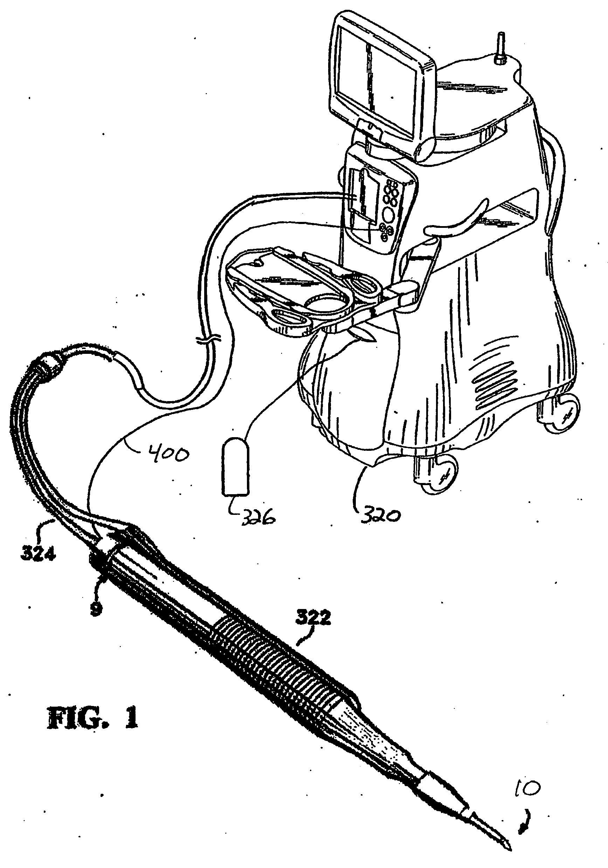

As best seen in FIG. 1, surgical console 320 suitable for use with the present invention may be any commercially available surgical control console such as the INFINITI^{®} Vision System available from Alcon Laboratories, Inc., Fort Worth, Texas. Console 320 is connected to handpiece 9 through irrigation line 322 and aspiration line 324, and the flow through lines 322 and 324 is controlled by the user, for example, via footswitch 326. Power is supplied to handpiece 9 through electrical cable 400.

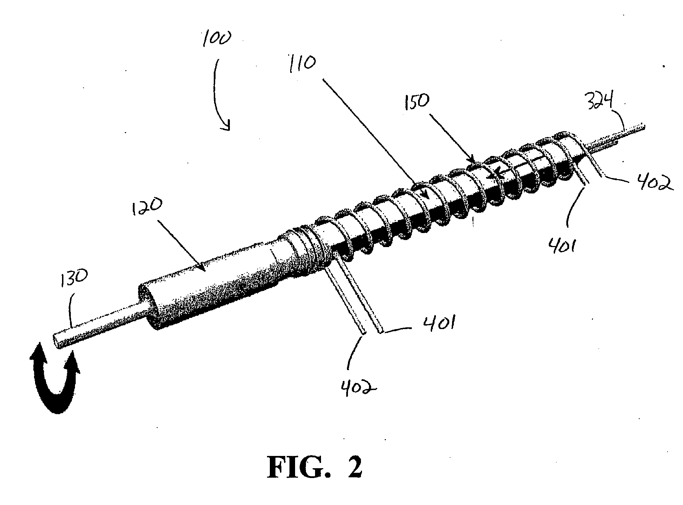

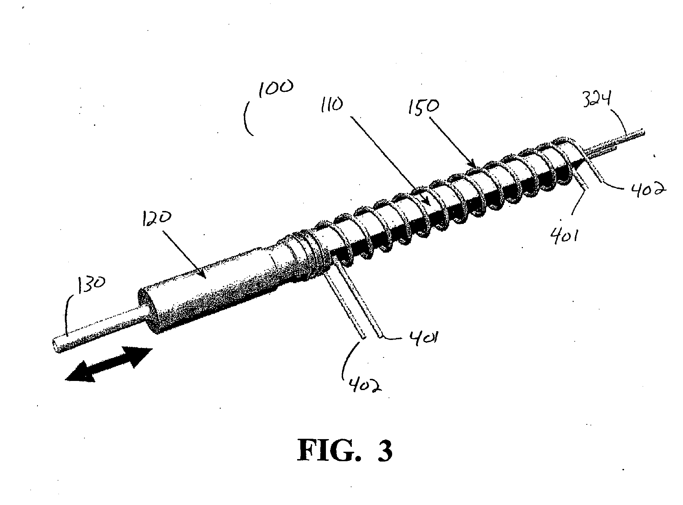

In a first embodiment of the present invention, best seen in FIGS. 2 and 3, handpiece 9 of the present invention may contain magnetostrictive core 100. Core 100 generally contains laminated magnetostrictive stack 110, attached at the distal end of stack 110 is acoustic impedance transformer, or horn, 120. Horn 120 is a body of metal of suitable shape and thickness necessary to convert the vibrations of stack 110 into longitudinal motion. Horn 120 has distal end 130 to which tip 10 is connected. Surrounding stack 110 is electrical coil 150. A drive signal is supplied to stack 110 and coil 150 through cable 400 and wires 401 and 402, respectively.

As best seen in FIG. 2, when a drive signal is passed through both coil 150 and stack 110, stack 110 moves in a twisting motion, consistent with the Wiedemann effect. Such a twisting motion causes horn 120, including distal end 130 and tip 10 to also move in a twisting or torsional manner. The extent of the amount of coil 150 covering stack 110 can be modified to control the magnitude and location of the twisting motion. In addition, the amplitude and frequency of the drive signal can be used to control the amplitude and frequency of the twisting motion.

In addition, as best seen in FIG. 3, when a drive signal is passed only through coil 150 and no drive signal is passed through stack 110, such coil-only excitation will produce only longitudinal movement in stack 110, with consequential longitudinal movement in horn 120, distal end 130 and tip 10. One skilled in the art will recognize that vibrating horn 120 in a continuous fashion or alternating between longitudinal and torsional motions may be desirable depending upon the desired surgical technique. This is accomplished by continuously passing a drive signal though coil 150 while continuously and/or episodically passing a drive signal through stack 110.

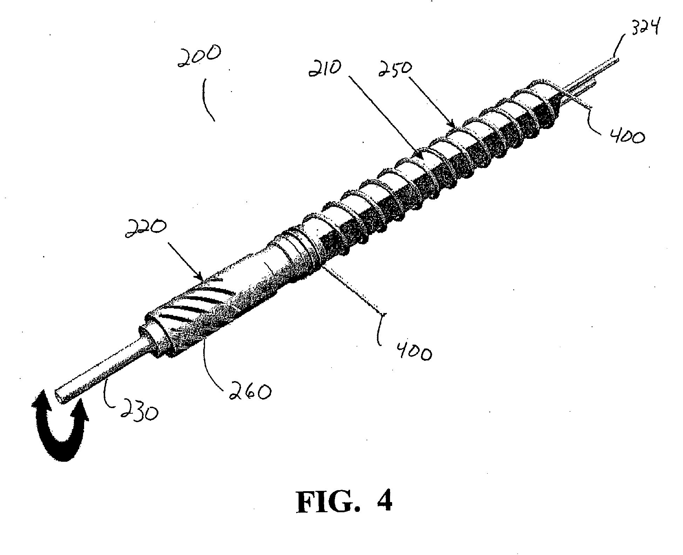

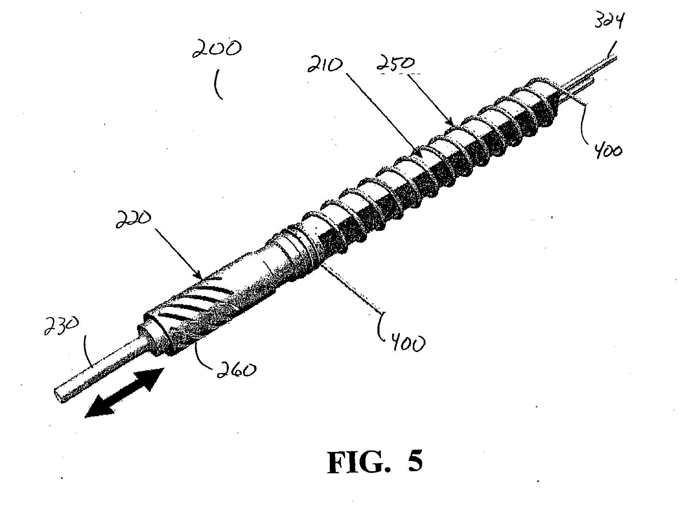

In a second embodiment of the present invention, best seen in FIGS. 4 and 5, handpiece 9 of the present invention may contain magnetostrictive core 200. Core 200 generally contains laminated magnetostrictive stack 210, attached at the distal end of stack 210 is acoustic impedance transformer, or horn, 220. Horn 220 is a body of metal of suitable shape and thickness necessary to convert the vibrations of stack 210 into longitudinal motion. Horn 220 has a series of parallel, angled slits 260 and distal end 230 to which tip 10 is connected. Surrounding stack 210 is electrical coil 250. Power is supplied to coil 250 through cable 400.

As best seen in FIG. 5, when a drive signal having a first frequency and amplitude, for example 30 Khz, is passed through coil 250, stack 210 moves with a longitudinal motion that imparts a twisting or torsional movement into horn 220 and distal end 230 because of the slits 260. As best seen in FIG. 3, when a drive signal having a second frequency and amplitude, for example 20 Khz, is passed through coil 250, stack 210 moves with a longitudinal motion, but because the longitudinal motion is not at the resonant frequency of slits 260, horn 220 and distal end 230 also vibrate longitudinally. One skilled in the art will recognize that vibrating horn 220 in a continuous fashion or alternating between longitudinal and torsional motions may be desirable depending upon the desired surgical technique.

One skilled in the art will recognize that core 200 may also be operated in a manner described above with respect to core 100.

While certain embodiments of the present invention have been described above, these descriptions are given for purposes of illustration and explanation. Variations, changes, modifications and departures from the systems and methods disclosed above may be adopted without departure from the scope of the present invention.

## Claims

1. An ultrasonic surgical handpiece capable of providing both longitudinal and torsional or twisting motion at a handpiece tip (10), comprising an acoustic impedance transformer, or horn (120,220), and a magnetorestrictive core (100,200), said magnetorestrictive core comprising:
(a) a laminated magnetorestrictive stack (110,210) coupled to the horn;
(b) an electric coil (150,250) surrounding the stack; and
(c) means (401, 402) adapted to supply a selected drive signal to the coil so as to cause longitudinal motion of the stack and the horn,
**characterized in that** said means are further adapted to supply a selected drive signal to the coil so as to cause longitudinal motion of the stack and the horn and to the stack so as to cause torsional motion of the stack and the horn.

2. The handpiece of claim 1, wherein the horn (220) has a plurality of slits (260).

3. The handpiece of claim 2, wherein the plurality of slits (260) is a series of parallel angled slits, sized and spaced so as to produce a torsional movement in the horn (220) on account of the resonant frequency of the slits in response to a drive signal supplied to the coil (250) having a first frequency, and longitudinal movement in the horn in response to a drive signal having a second frequency.

4. The handpiece of claim 1, wherein;
(a) a first drive signal is passed through the coil (150) to cause longitudinal movement of the stack (110) and the horn (120); and
(b) a second drive signal is passed continuously and/or episodically through the stack to cause torsional movement of the stack (110) and the horn (120).

5. The handpiece of claim 2 or claim 3, wherein;
(a) a first drive signal having a first frequency is passed through the coil (250) so as to produce torsional movement in the horn (220);
(b) a second drive signal having a second frequency is passed continuously and/or episodically through the coil (250) so as to produce longitudinal movement in the horn (220); and
(c) a third drive signal is passed continuously and/or episodically through the stack (210).

## Patentansprüche

1. Chirurgisches Ultraschall-Handstück, das in der Lage ist, sowohl eine Längs- als auch Torsions- oder Dreh-Bewegung an einer Spitze (10) des Handstücks zu ermöglichen, mit einem akustischen Impedanzwandler oder Horn (120, 220) und einem magnetostriktiven Kern (100, 200), der
(a) einen magnetostriktiven Schicht-Stapel (110, 210), der mit dem Horn gekuppelt ist;
(b) eine elektrische Spule (150, 250), die den Stapel umgibt; und
(c) Mittel (401, 402) enthält, die eingerichtet sind, um ein ausgewähltes Treibersignal an die Spule zu liefern, um eine Längs-Bewegung des Stapels und des Horns zu bewirken,
**dadurch gekennzeichnet, dass** die Mittel weiters eingerichtet sind, um ein ausgewähltes Treibersignal an die Spule, um eine Längs-Bewegung des Stapels und des Horns zu bewirken, und an den Stapel zu liefern, um eine Torsions-Bewegung des Stapels und des Horns zu bewirken.

2. Handstück nach Anspruch 1, wobei das Horn (220) eine Mehrzahl von Schlitzen (260) aufweist.

3. Handstück nach Anspruch 2, wobei die Mehrzahl von Schlitzen (260) eine Reihe von parallelen winkeligen Schlitzen ist, deren Größe und Abstand so gewählt ist, dass aufgrund der Resonanzfrequenz der Schlitze in Reaktion auf ein der Spule (250) zugeführtes Treibersignal mit einer ersten Frequenz eine Torsions-Bewegung im Horn (220) und in Reaktion auf ein Treibersignal mit einer zweiten Frequenz eine Längs-Bewegung im Horn erzeugt wird.

4. Handstück nach Anspruch 1, wobei:
(a) ein erstes Treibersignal die Spule (150) durchläuft, um eine Längs-Bewegung des Stapels (110) und des Horns (120) zu bewirken; und
(b) ein zweites Treibersignal den Stapel kontinuierlich und/oder fallweise, durchläuft, um eine Torsions-Bewegung des Stapels (110) und des Horns (120) zu bewirken.

5. Handstück nach Anspruch 2 oder 3, wobei:
(a) ein erstes Treibersignal mit einer ersten Frequenz die Spule (250) durchläuft, um eine Torsions-Bewegung im Horn (220) zu bewirken;
(b) ein zweites Treibersignal mit einer zweiten Frequenz die Spule (250) kontinuierlich und/oder fallweise durchläuft, um eine Längs-Bewegung im Horn (220) zu bewirken; und
(c) ein drittes Treibersignal den Stapel (210) kontinuierlich und/oder fallweise durchläuft.

## Revendications

1. Pièce à main chirurgicale à ultrasons capable de réaliser à la fois un mouvement longitudinal et de rotation ou de torsion à une extrémité de pièce à main (10), comprenant un transformateur d'impédance acoustique, ou cornet (120, 220), et un noyau magnétostrictif (100, 200), ledit noyau magnétostrictif comprenant :
(a) une pile magnétostrictive (110, 210) stratifiée accouplée au cornet ;
(b) une bobine électrique (150, 250) entourant la pile ; et
(c) des moyens (401, 402) adaptés pour appliquer un signal de commande sélectionné à la bobine de manière à provoquer un mouvement longitudinal de la pile et du cornet,
**caractérisée en ce que** lesdits moyens sont en outre adaptés pour appliquer un signal de commande sélectionné à la bobine de manière à provoquer un mouvement longitudinal de la pile et du cornet et à la pile de manière à provoquer un mouvement de torsion de la pile et du cornet.

2. Pièce à main selon la revendication 1, dans laquelle le cornet (220) comporte une pluralité de fentes (260).

3. Pièce à main selon la revendication 2, dans laquelle la pluralité de fentes (260) consiste en une série de fentes inclinées parallèles, dimensionnées et espacées de manière à produire un mouvement de torsion dans le cornet (220) à cause de la fréquence de résonance des fentes en réponse à un signal de commande délivré à la bobine (250) ayant une première fréquence, et un mouvement longitudinal dans le cornet en réponse à un signal de commande ayant une deuxième fréquence.

4. Pièce à main selon la revendication 1, dans laquelle :
(a) un premier signal de commande est appliqué à travers la bobine (150) pour provoquer un mouvement longitudinal de la pile (110) et du cornet (120) ; et
(b) un deuxième signal de commande est appliqué en continu et/ou de manière épisodique à travers la pile pour provoquer un mouvement de torsion de la pile (110) et du cornet (120).

5. Pièce à main selon la revendication 2 ou la revendication 3, dans laquelle :
(a) un premier signal de commande ayant une première fréquence est appliqué à travers la bobine (250) de manière à produire un mouvement de torsion dans le cornet (220) ;
(b) un deuxième signal de commande ayant une deuxième fréquence est appliqué en continu et/ou de manière épisodique à travers la bobine (250) de manière à produire un mouvement longitudinal dans le cornet (220) ; et
(c) un troisième signal de commande est appliqué en continu et/ou de manière épisodique à travers la pile (210).
